# EUROPEAN PATENT APPLICATION

(11) **EP 4 226 925 A1**
(43) Date of publication of application: **16.08.2023**
(21) Application number: 21877769.6
(22) Date of filing: 08.10.2021
(51) Int. Cl.: A61K 31/455, A61K 9/107, A61K 47/64, A61P 3/02, A61P 9/12, A61P 43/00, C08G 69/40, C08G 69/48

(54) **NICOTINAMIDE-ENCAPSULATING MICELLES, AND PREGNANCY-INDUCED HYPERTENSION SYNDROME THERAPY COMPOSITION CONTAINING NICOTINAMIDE-ENCAPSULATING MICELLES**

(30) Priority: 09.10.2020 JP 2020171414
(71) Applicant: The University of Tokyo, Bunkyo-ku, Tokyo 113-8654 (JP); ASKA Pharmaceutical Co., Ltd., Tokyo 108-8532 (JP)
(72) Inventor: CABRAL, Horacio, Tokyo 113-8654 (JP); MIYAZAKI, Takuya, Tokyo 113-8654 (JP); CHEN, Pengwen, Tokyo 113-8654 (JP); KAWASHIMA, Naoya, Fujisawa-shi, Kanagawa 251-8555 (JP)
(74) Representative: advotec.
(86) International application number: PCT/JP2021/037453
(87) International publication number: WO 2022/075470

(57) **Abstract**

The presemt invention addresses the problem of providing a pregnancy-induced hypertension syndrome therapy composition having controlled placental permeability. Provided are nicotinamide-encapsulating micelles having a particle diameter of 25 - 100 nm. Controlling the particle diameter of the nicotinamide-encapsulating micelles enables control of placental permeability so as to make it possible to accumulate nicotinamide-encapsulating micelles in a placental while suppressing transfer to a fetus. Administering the nicotinamide-encapsulating micelles brings about a superior effect of reducing blood pressure during a pregnancy-induced hypertension syndrome.

## Description

### FIELD

The present invention relates to a composition for treatment of gestational hypertension syndrome with reduced placental permeability.

### BACKGROUND

Hypertension occurring after the 20th week of pregnancy is referred to as gestational hypertension. Blood pressure generally falls slightly after establishment of pregnancy, gradually rising from the 20th week thereafter until birth. When hypertension occurs within 20 weeks after pregnancy, the condition is referred to as "pregnancy with hypertension". Gestational hypertension is sometimes associated with proteinuria and edema. The symptoms accompanying hypertension during pregnancy are collectively referred to as gestational hypertension syndrome. Gestational hypertension syndrome is classified into pregnancy hypertension, preeclampsia and superimposed preeclampsia, with more serious gestational hypertension syndrome being associated with complications such as eclampsia, cerebral hemorrhage, impaired liver function, HELLP syndrome, impaired renal function, neuropathy, blood clotting disorder, fetal growth retardation and placental abruption.

When gestational hypertension syndrome has been diagnosed, antihypertensive drugs are administered when no improvement has been seen after a course of rest and alimentary therapy. However, antihypertensive drugs can potentially cause fetal hypoxia and malnutrition, leading to maldevelopment of the fetus. Antihypertensive drugs must therefore be administered with care under the supervision of a physician. Many drugs are contraindicated for pregnancy due to problems of teratogenicity, and the effects of the drugs that can be used are often insufficient.

When an adequate therapeutic effect is not obtained even with administration of an antihypertensive drug, and the life of either the mother or fetus is at risk, it may be decided to interrupt the pregnancy by Caesarean section or induced labor. The symptoms of gestational hypertension syndrome usually subside after interruption of pregnancy, but in serious cases the symptoms such as hypertension and proteinuria may continue after birth. While advances in neonatal intensive care have improved survival rates for underweight fetuses resulting from premature birth, after-effects can occur during subsequent growth, and it is therefore desirable to establish treatment methods for gestational hypertension syndrome.

Nicotinamide is an inhibitor of the cyclic ADP ribose synthase ADP-ribosylcyclase, and is known to block vasoconstriction due to endothelin-1 (ET-1) or angiotensin II (AngII). Nicotinamide has been reported to have no teratogenicity or carcinogenic effects (NPL 1: Diabetologia (2000) vol. 43(11), 1337-45). Nicotinamide is also administered as a vitamin during pregnancy, and is currently being developed as a therapeutic or preventive agent for gestational hypertension syndrome (PTL 1: Japanese Unexamined Patent Publication No. 2015-30721).

### [CITATION LIST]

### [PATENT LITERATURE]

[PTL 1] Japanese Unexamined Patent Publication No. 2015-30721

### [NON PATENT LITERATURE]

[NPL 1] Diabetologia (2000)vol. 43(11), 1337- 45
[NPL 2] Macromol. Biosci. (2020) (20) 1900161
[NPL 3] H. Cabral et. al., Nat. Nanotech. 6 (2011) 815-823
[NPL 4] K. Shintaku et. al., Drug Metab. Dispos. 37 (2009) 962
[NPL 5] J. R. Huston et. al., Clin. Pharmacol. Ther. 90 (2011) 67
[NPL 6] M. Nagai et. al., Drug Metab. Dispos. 41 (2013) 2124
[NPL 7] F. Li et. al., PNAS (2016), vol. 113, No. 47 13450-13455

### SUMMARY

### [TECHNICAL PROBLEM]

Therapeutic agents containing nicotinamide are being actively developed for gestational hypertension syndrome. Nicotinamide has been reported to lower blood pressure during pregnancy at doses confirmed to be safe for humans in terms of teratogenicity or carcinogenicity. When administered during pregnancy, however, it is necessary for transplacental effects on the fetus to be evaluated in a more multifaceted manner to include possible neurotoxic effects, in addition to teratogenicity and carcinogenicity. It is therefore desirable to develop a drug that has reduced placental transmission while being targeted to the site of action.

### [SOLUTION TO PROBLEM]

As a result of much research on the placental permeability of drugs, the present inventors have found that placental permeability can be controlled by modifying the structures of hydrophilic polymer or hydrophobic polymer nanoparticles to control their sizes. It was found that limiting the placental permeability allows accumulation of the nanoparticles in the placenta, and the present invention has been completed based on this finding. Specifically, the present invention relates to the following:
[1] Nicotinamide-encapsulating micelles formed from a polyether-polyamino acid block copolymer, wherein nicotinamide is bonded to an amino acid side chain, and wherein the particle size is 25 to 100 nm as measured by dynamic light scattering.
[2] The micelles according to [1] above, wherein the polyether-polyamino acid block copolymer comprises lysine as an amino acid block.
[3] The micelles according to [1] or [2] above, wherein the polyether-polyamino acid block copolymer is a polyethylene glycol-polyamino acid block copolymer.
[4] The micelles according to any one of [1] to [3] above, wherein the nicotinamide is bonded to the amino acid side chain via a carboxymethylmaleic anhydride reactive group.
[5] The micelles according to any one of [1] to [4] above, wherein the polyether-polyamino acid block copolymer is a compound represented by the following formula: 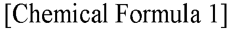 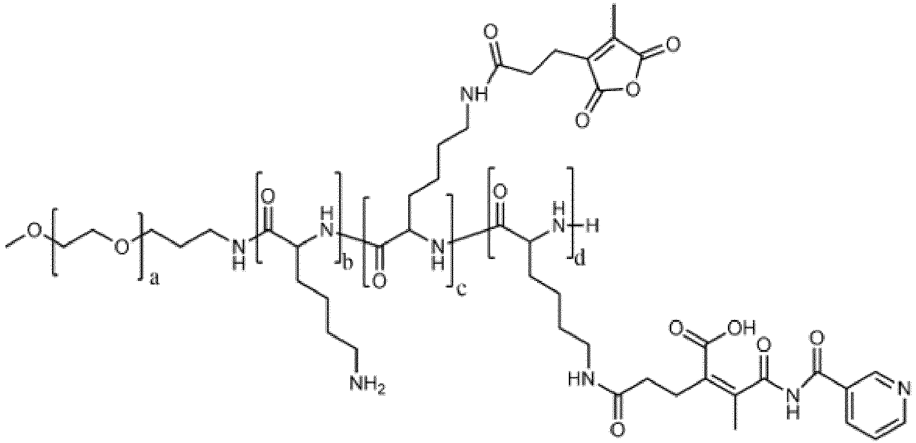 (wherein a = 45 to 2000, b = 0 to 20, c = 0 to 39 and d = 1 to 40).
[6] The micelles according to any one of [1] to [5] above, wherein the particle size of the nicotinamide-encapsulating micelles is 30 to 50 nm.
[7] A composition containing micelles according to any one of [1] to [6] above.
[8] The composition according to [7] above, wherein the composition is a composition for treatment or prevention of hypertension during pregnancy.
[9] The composition according to [8] above, wherein the hypertension during pregnancy is gestational hypertension syndrome and includes miscarriage, premature birth and/or intrauterine fetal growth retardation caused by gestational hypertension syndrome.
[10] The composition according to any one of [7] to [9] above, wherein the migration to the fetus is reduced.
[11] The composition according to any one of [7] to [10] above, wherein the composition is a placenta-targeted composition.
[12] A method for producing nicotinamide-encapsulating micelles for treatment of pregnancy hypertension, wherein the method includes:
   reacting nicotinamide with a polyether-polyamino acid block copolymer that includes a carboxymethylmaleic anhydride-reactive group on an amino acid side chain;
   purifying the nicotinamide-loaded polyether-polyamino acid block copolymer;
   dissolving the nicotinamide-loaded polyether-polyamino acid block copolymer in a polar solvent; and
   ultrafiltration using a dialysis membrane with a molecular cutoff of 3500 to 16,000 to obtain micelles having a particle size of 25 to 100 nm as measured by dynamic light scattering.
[13] The method according to [12] above, wherein the polyether-polyamino acid block copolymer that includes a carboxymethylmaleic anhydride-reactive group on an amino acid side chain is represented by the following formula: 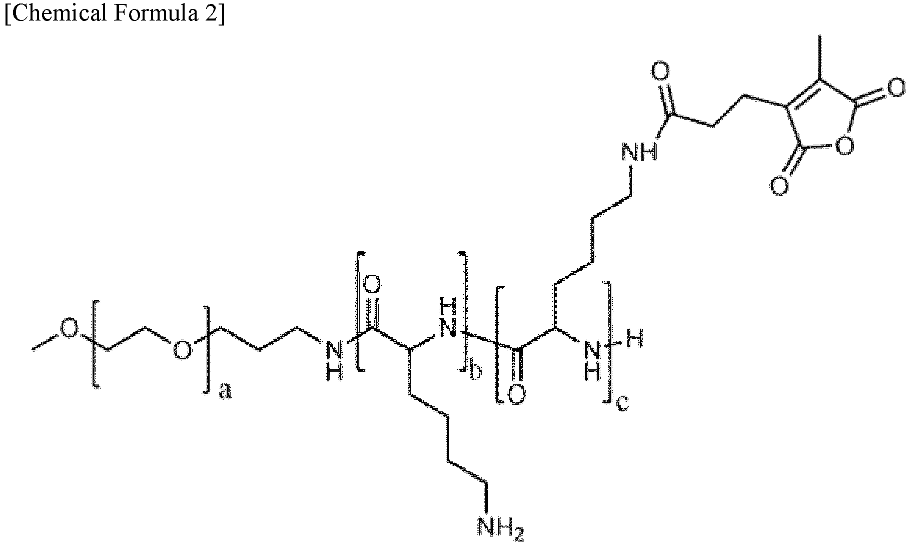 (wherein a = 45 to 2000, b = 0 to 20 and c = 1 to 60).
[14] The micelles according to any one of [1] to [6] above, which is for use in treatment or prevention of hypertension during pregnancy.
[15] The micelles according to any one of [1] to [6] above, which is for use in treatment or prevention of gestational hypertension syndrome, miscarriage or premature birth, and/or intrauterine fetal growth retardation.
[16] The micelles according to [14] or [15] above, which mediate reduction of migration into the fetus.
[17] The micelles according to [14] or [15] above, which mediate accumulation in the placenta.
[18] A method for treatment or prevention of hypertension in a subject during pregnancy,
   wherein the method includes administering micelles according to any one of [1] to [6] to the subject.
[19] A method for treatment or prevention of gestational hypertension syndrome, miscarriage or premature birth, and/or intrauterine fetal growth retardation in a subject suffering from hypertension during pregnancy,
   wherein the method includes administering micelles according to any one of [1] to [6] above to the subject.
[20] The method according to [18] or [19] above, which mediates reduction of migration into the fetus.
[21] The method according to [18] or [19] above, which mediates accumulation in the placenta.
[22] The use of micelles according to any one of [1] to [6] above for production of a drug for treatment or prevention of hypertension during pregnancy.
[23] The use of micelles according to any one of [1] to [6] above for production of a drug for treatment or prevention of gestational hypertension syndrome, miscarriage or premature birth, and/or intrauterine fetal growth retardation.
[24] The use according to [22] or [23] above, which mediates reduction of migration into the fetus.
[25] The use according to [22] or [23] above, which mediates accumulation in the placenta.

### [ADVANTAGEOUS EFFECTS OF INVENTION]

The nicotinamide-encapsulating micelles of the invention have reduced placental permeability and inhibited migration of nicotinamide to the fetus. When incorporated into a cell by endocytosis, the nicotinamide is released into the low pH environment of the endoplasmic reticulum, allowing it to act on the cell by which it has been endocytosed. Micellation causes increased retentivity of nicotinamide in the blood, resulting in increased accumulation in the placenta. Micellation of nicotinamide also increases the blood pressure lowering effect in a subject with gestational hypertension syndrome.

### BRIEF DESCRIPTION OF DRAWINGS

Fig. 1A shows NMR data for polyethylene glycol-poly(lysine) block copolymer (PEG-p(Lys)). Fig. 1B shows GPC data.
Fig. 2 shows NMR data for polyethylene glycol-poly(lysine-carboxymethylmaleic anhydride) block copolymer (PEG-p(Lys-CDM)).
Fig. 3 shows NMR data for nicotinamide bonded polyethylene glycol-polyamino acid block copolymer (PEG-p(Lys-CDM-NA)).
Fig. 4 shows NMR data for nicotinamide-encapsulating micelles (PEG-p(Lys-CDM-NA) micelles).
Fig. 5 shows release amounts of nicotinamide in an external solution, after dialysis of nicotinamide-encapsulating micelles against the external solution under physiological conditions (pH 7.4) and intravesicular conditions (pH 5.5).
Fig. 6 shows the results of examining the placental permeability of nicotinamide and nicotinamide-encapsulating micelles, using a human placental perfusion model.
Fig. 7 shows blood retentivity of nicotinamide and nicotinamide-encapsulating micelles after injection into the caudal vein of normal pregnant mice.
Fig. 8 shows placental accumulation (1) and fetal accumulation (2) of nicotinamide and nicotinamide-encapsulating micelles, after injection into the caudal vein of normal pregnant mice.
Fig. 9 shows the results of examining the therapeutic effect of nicotinamide and nicotinamide-encapsulating micelles on pregnancy hypertension in a pregnancy hypertension mouse model.
Fig. 10 shows placental accumulation (1) of nicotinamide and nicotinamide-encapsulating micelles, after injection into the caudal vein of normal pregnant mice, and placental accumulation (2) of nicotinamide and nicotinamide-encapsulating micelles after injection into the caudal vein of hypertension model mice.
Fig. 11 is a photograph showing degree of accumulation of polymer micelles in a placenta and fetus.
Fig. 12 is a graph showing the placental permeability of drugs in a human placental perfusion model. Fig. 12A shows the results for oxaliplatin, 30 nm DACH-platin encapsulating micelles, 70 nm DACH-platin encapsulating micelles and 8-armPEG. Fig. 12B shows the results for 10 nm, 20 nm and 30 nm PEG-coated gold nanoparticles.

### DESCRIPTION OF EMBODIMENTS

Unless otherwise specified, the terms used herein throughout are understood to have the same meanings as used in the relevant field. Unless otherwise defined, therefore, all of the technical and scientific terms used herein have the same meanings as generally understood by a person skilled in the technical field of the present invention. The numerical ranges specified herein include their upper and lower limits.

### [Definitions]

### [Nicotinamide]

Nicotinamide is the compound pyridine-3-carboxyamide (IUPAC name). Nicotinamide, also known as niacinamide, is a water-soluble vitamin of the vitamin B group. The compound nicotinic acid (niacin), or vitamin B₃, is converted to nicotinamide in the liver, and its function as a vitamin is basically equivalent to that of nicotinamide. Nicotinamide is a powerful inhibitor of the cyclic ADP ribose synthase ADP-ribosylcyclase, and is known to block vasoconstriction due to ET-1 or AngII. Nicotinamide has no teratogenicity or carcinogenicity at normal doses, and is used as a safe drug or food (vitamin) for pregnancy in cases of pellagra (nicotinic acid deficiency) or inadequate nicotinic acid ingestion.

### [Gestational hypertension syndrome]

Gestational hypertension syndrome is a pathological condition that is primarily hypertension but also causes edema and proteinuria, and according to the Japan Society of Obstetrics and Gynecology it is defined as "hypertension from the 20th week of pregnancy up until the 12th week postpartum, or proteinuria with hypertension, where the symptoms are not due to accidental complications of pregnancy".

### [Micelles]

According to the invention, the term "micelles" refers to aggregates of numerous molecules including hydrophilic portions and hydrophobic portions associating by hydrophobic interaction. A polymer that includes hydrophilic portions and hydrophobic portions forms micelles with the hydrophilic portions on the outer side and the hydrophobic portions on the inner side. According to the invention, a polyether-polyamino acid block copolymer functions as the molecule with hydrophilic portions and hydrophobic portions to form the micelles.

### [Block copolymer]

A block copolymer is a polymer wherein multiple different monomers polymerize in order forming multiple regions. The number of regions may be selected as desired, but for the purpose of the invention the block copolymer is a diblock copolymer formed of polyether regions and polyamino acid regions. The polyether-polyamino acid block copolymer includes a hydrophilic portion formed of a polyether and a hydrophobic portion formed of a polyamino acid which is hydrophobic due to the nature of its side chain. A drug can be introduced into the polyamino acid side chains, with the side chains where the drug has been introduced becoming hydrophobic to form the hydrophobic cores of the micelles. Polyethylene glycol, polypropylene glycol or polybutylene glycol may be selected as the polyether from the viewpoint of obtaining sufficient hydrophilicity, with polyethylene glycol being most preferred.

### [Placenta]

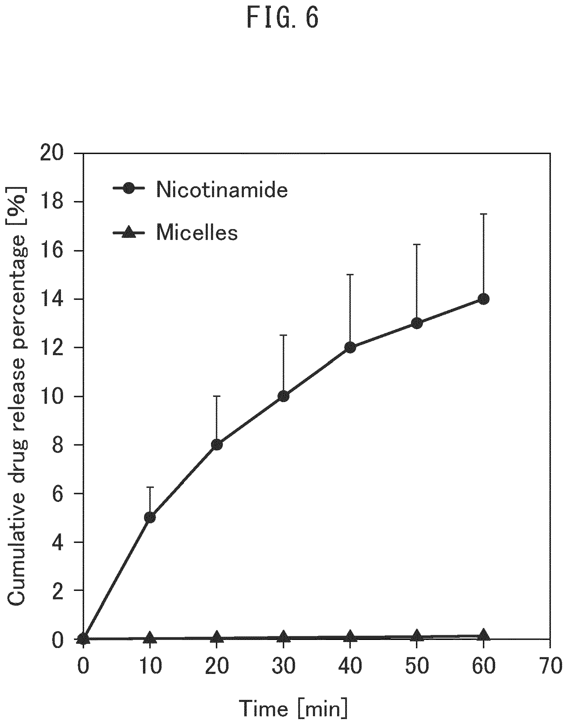

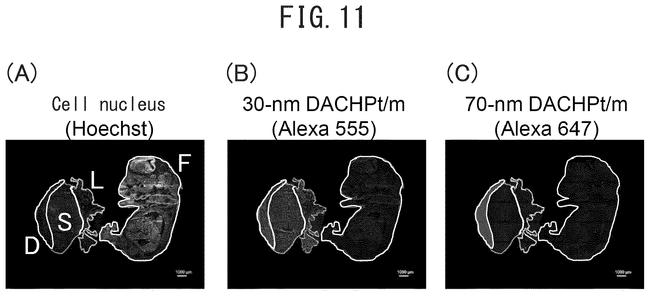

The placenta is the organ connecting mother and fetus. Gas exchange of oxygen and carbon dioxide, as well as absorption of nutrients and discharge of waste products, takes place via the placenta. Hormones such as progesterone and estrogen are also secreted from the placenta and contribute to continuation of pregnancy. Blood pressure regulating factors such as vasodilating factors and antagonists are also secreted from the placenta, and their action is believed to be the cause of gestational hypertension syndrome. The placenta is composed of a maternal decidua (D) layer and a fetal labyrinth (L) layer, and a spongiotrophoblast (S) layer between the D layer and L layer. Since blood substances do not travel freely to and from the maternal and fetal sides through the placenta, it is also known as the placental barrier. The particle sizes of micelles can be controlled in order to modify their placental permeability. As a non-limitative example, micelles with particle sizes of 30 nm can migrate through to the D layer and S layer but are inhibited from migrating to the fetus. Micelles with particle sizes of 70 nm can only migrate up to the D layer (Fig. 11). Some of the micelles that do not pass through the placenta will accumulate in the placenta. The nicotinamide-encapsulating micelles of the invention cannot migrate through the human placenta to the fetus side, whereas non-micellated nicotinamide passes through human placenta to reach the fetus (Fig. 6). Micelles that have accumulated in the placenta are incorporated into the cells of the placenta by endocytosis, and act on them. According to another aspect of the invention, therefore, a composition containing micelles of the invention can be used as a pharmaceutical composition which is targeted to the placenta, or serves to reduce fetal drug toxicity or to limit passage through the placental barrier.

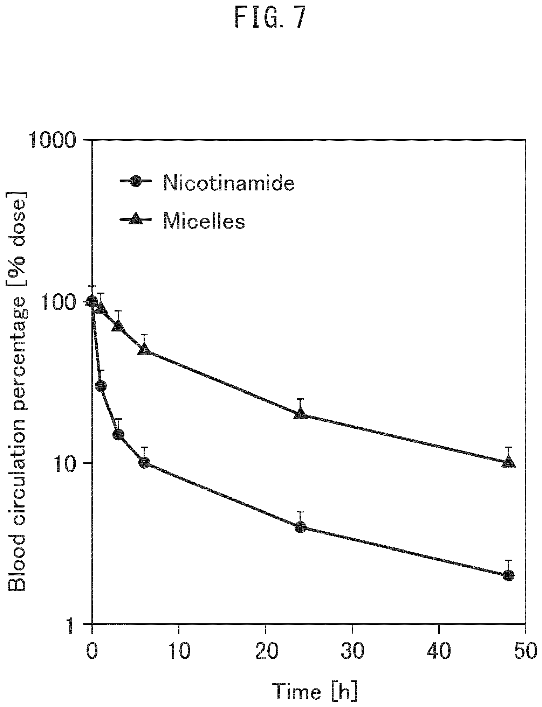

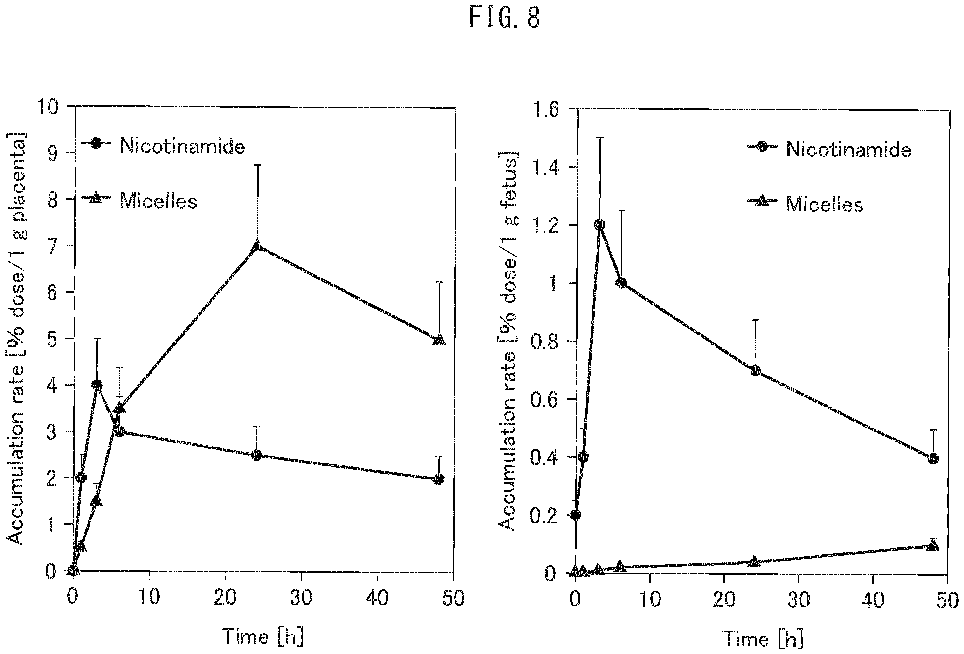

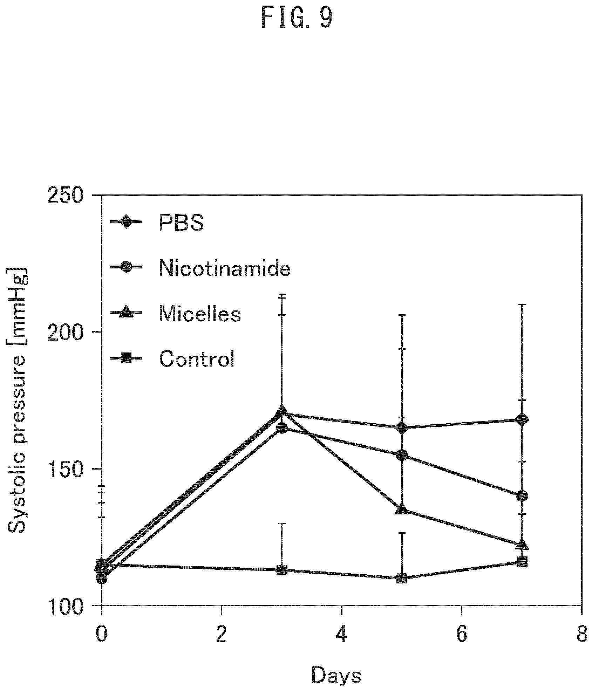

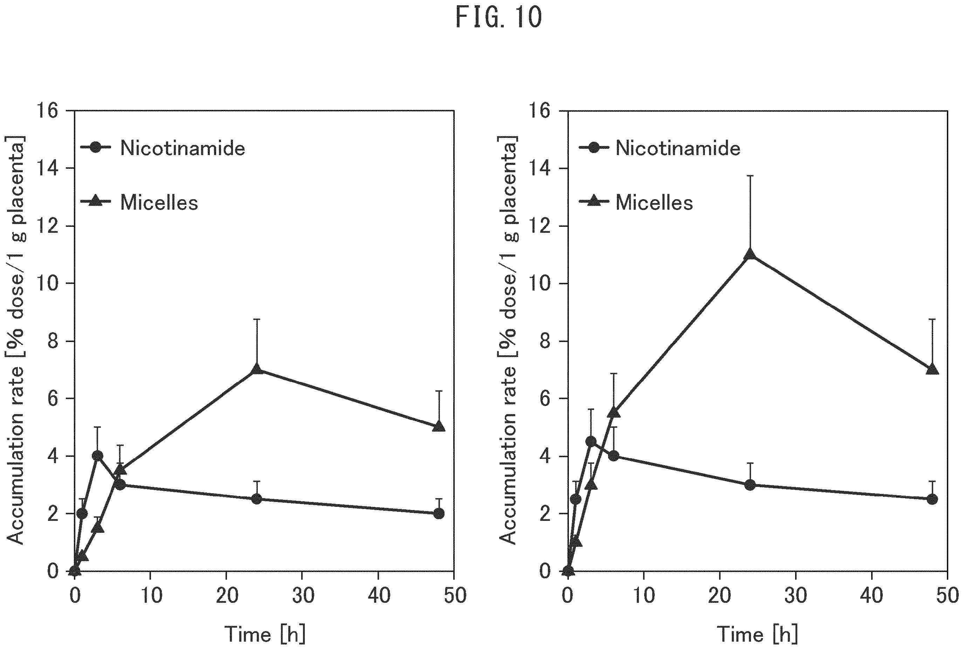

The invention relates to nicotinamide-encapsulating micelles formed from a nicotinamide-bonded polyether-polyamino acid block copolymer. One feature of the invention is that the particle sizes of the micelles are 25 to 200 nm as measured by dynamic light scattering. From the viewpoint of the placental permeability, the particle sizes are larger than 25 nm, preferably 30 nm or larger, and even more preferably 35 nm or larger. From the viewpoint of avoiding the hepatic reticuloendothelial system, the particle sizes are generally 200 nm or smaller and preferably 100 nm or smaller, while from the viewpoint of targeting the placenta they are more preferably 75 nm or smaller. Particles of such size have low placental permeability and can lower the effect on the fetus. The polydispersity index (PDI) for the particle sizes in the composition of the invention is preferably 0.2 or lower and more preferably 0.15 or lower. Retentivity in the blood is improved with nicotinamide-encapsulating micelles as compared to nicotinamide (Fig. 7). Whereas nicotinamide is rapidly absorbed after administration, nicotinamide-encapsulating micelles, having higher blood retentivity, can exhibit a more long-lasting drug effect. In addition, the accumulation of nicotinamide in tissues peaks within about 10 minutes following administration, the accumulation being in both placental and fetal tissue, whereas after administration of nicotinamide-encapsulating micelles, accumulation in the placenta peaks starting from 20 minutes, with a greater amount of accumulation as well, while accumulation in the fetus is inhibited (Fig. 8). In a hypertension model, the amount of accumulation in the placenta is even higher compared to a normal model, resulting in activity at the active site of the placenta to rapidly lower blood pressure (Fig. 10). In addition to acting on the vascular endothelium to lower blood pressure, nicotinamide also acts at the placenta as one of its active sites, inhibiting production of sFlt1 from the placenta (NPL 7:PNAS (2016), vol. 113, No. 47 13450-13455). The factor sFlt1 is a vascular growth factor-inhibiting protein produced by the placenta which contributes to pregnancy hypertension. The blood pressure lowering effect obtained by administering nicotinamide-encapsulating micelles to pregnancy hypertension model mice is actually higher than the blood pressure lowering effect obtained by administering nicotinamide, suggesting accumulation and activity at the desired nicotinamide active sites (Fig. 9).

Micelles formed from a polyether-polyamino acid block copolymer have a pH-dependent drug-release property. This drug release property varies depending on the number of polyether units in the block copolymer, the types and numbers of amino acid units, and the type and amount of the encapsulated drug. With the nicotinamide-encapsulating micelles of the invention, approximately 70% of the nicotinamide is released by 24 hours at pH 5.5, compared to the approximate 10% release of nicotinamide by 24 hours at pH 7.4, which is the physiological pH in blood. The micelles are incorporated into cells by endocytosis in the body, then being disposed in the endoplasmic reticulum. The pH of the endoplasmic reticulum is lower than physiological pH, and normally about pH 5.5. When the nicotinamide-encapsulating micelles of the invention are incorporated into cells and disposed in the endoplasmic reticulum, they release the drug which acts directly inside the cells.

The polyamino acid portion of the polyether-polyamino acid block copolymer used for the invention can be selected as desired from the viewpoint of bondability with the nicotinamide to be encapsulated. Lysine may be used, as one example. From the viewpoint of facilitating production it is preferred to use a single type of amino acid, but a plurality of different amino acids may also be used. The dicarboxylic anhydride reactive group can be bonded to the amine group of lysine to allow further bonding of nicotinamide. In the formulas provided herein, the carboxylic anhydride reactive groups are shown as carboxymethylmaleic anhydride (CDM) (IUPAC name: 2,5-dihydro-4-methyl-2,5-dioxo-3-furanpropanoic acid), but other carboxylic anhydride reactive groups may be used instead. One example of a block copolymer to form the nicotinamide-encapsulating micelles of the invention is a compound represented by the following formula (I) or (II): 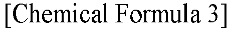 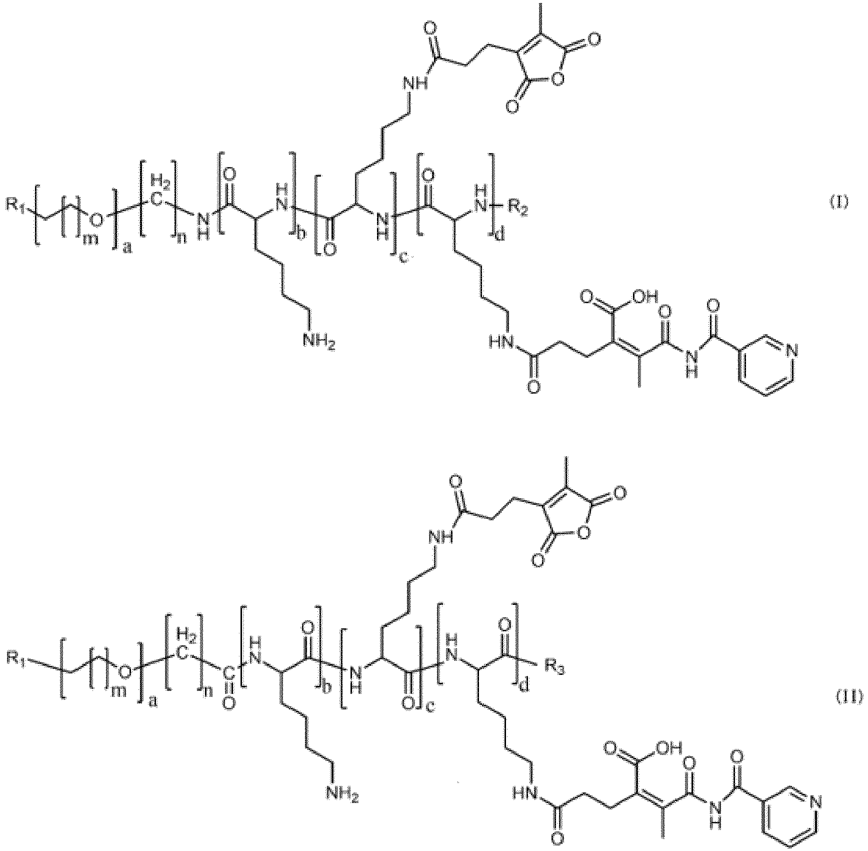 (where:
R₁ is the terminal group of the polyether,
R₂ and R₃ represent the terminal groups of the respective polyamino acids,
n = 1 to 5,
m = 1 to 3,
a = 45 to 2000,
b = 0 to 20,
c = 0 to 39, and
d = 1 to 40).

The polymers of formula (I) and formula (II) differ only in the orientation of the polyamino acid, by modification of the group connecting the hydrophilic polyether portion and the hydrophobic polyamino acid portion. Any one of these copolymers may be used since they have similar copolymer properties. Any copolymer of one orientation referred to herein also includes any of the copolymers with other orientations.

The polyether-polyamino acid block copolymers before introduction of nicotinamide are represented by the following formulas: 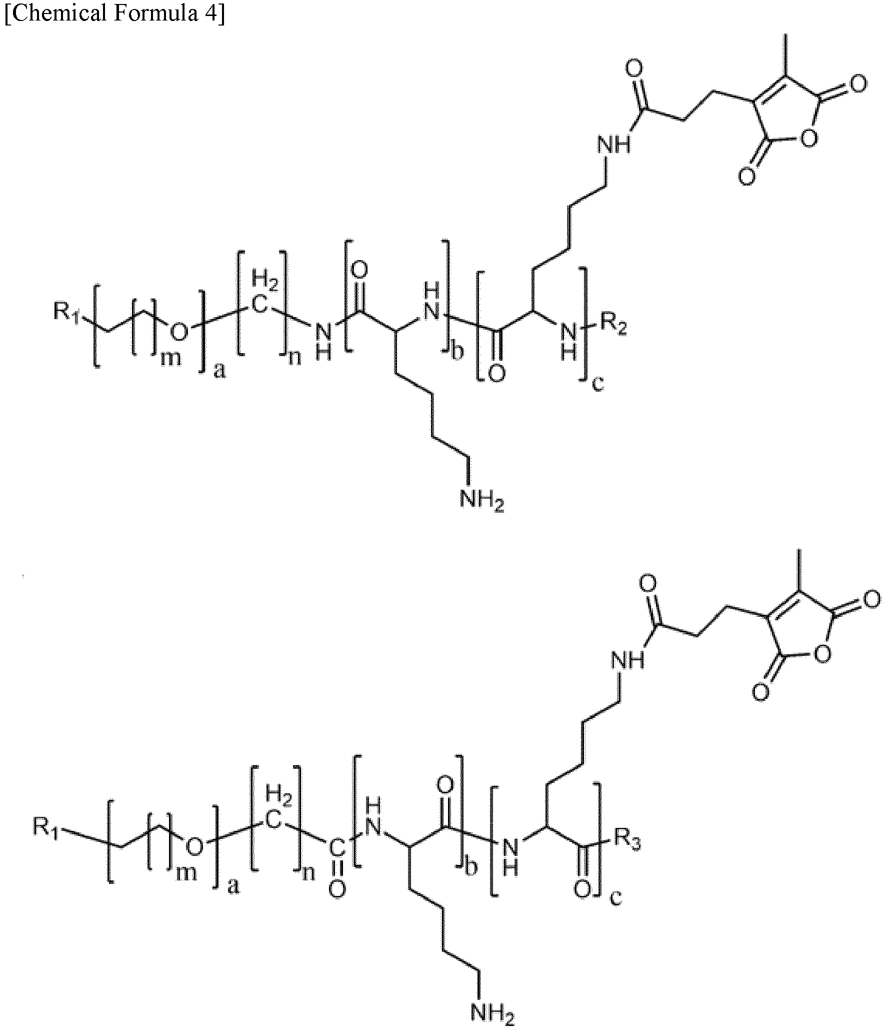 (where:
R₁ is the terminal group of the polyether,
R₂ and R₃ represent the terminal groups of the respective polyamino acids,
n = 1 to 5,
m = 1 to 3,
a = 45 to 2000,
b = 0 to 20, and
c = 1 to 60).

The group R₁ is the terminal group of the polyether, and it may be any group commonly used in copolymers, examples of which include hydrogen atoms, hydroxyl, or unsubstituted or substituted straight-chain or branched C₁₋₁₂ alkyl or C₁₋₁₂ alkoxy groups.

The group R₂ is the terminal group of the polyamino acid, and it may also be any group commonly used in copolymers, examples of which include hydrogen atoms, protecting groups, hydrophobic groups and polymerizable groups.

The group R₃ is the terminal group of the polyamino acid, and it may likewise be any group commonly used in copolymers, examples of which include hydroxyl, oxybenzyl and - NH-(CH₂)ₘ-X groups, or the initiator residue, where "m" is an integer of 1 to 5, and X is an amine compound residue that includes one or more primary, secondary or tertiary amines or a quaternary ammonium salt, or a non-amine compound residue.

From the viewpoint of micelle formation, the number of polyether repeats "a" in the polyether-polyamino acid block copolymer is preferably 45 or greater and more preferably 200 or greater, while from the viewpoint of controlling the micelle sizes it is preferably 2000 or less and more preferably 500 or less.

The number of non-CDM-bonded lysine residues "b" is determined based on the reaction rate between CDM and the amino acid side chain. The number of non-CDM-bonded lysine residues can be reduced by using an excess amount of CDM with respect to the polymer. From the viewpoint of increasing the amount of nicotinamide in the nicotinamide-bonded polyether-polyamino acid block copolymer, "b" is preferably 20 or smaller and more preferably 5 or smaller.

The number of non-nicotinamide-bonded, CDM-bonded lysine residues "c" is the number of lysine residues that are not bonded with nicotinamide but are bonded with CDM. The number of non-nicotinamide-bonded, CDM-bonded lysine residues "c" is determined based on the reaction rate between CDM and nicotinamide in the nicotinamide-bonded polyether-polyamino acid block copolymer as the final product, but it is preferably lower from the viewpoint of loading nicotinamide, with 39 or less being preferred, and usually 10 or less or 5 or less being more preferred. It is most preferably 0, in which case nicotinamide is loaded onto all of the CDM-bonded lysines present in the polyether-polyamino acid block copolymer. The number of non-nicotinamide-bonded, CDM-bonded lysine residues "c" in the polyether-polyamino acid block copolymer before introduction of nicotinamide, as the intermediate product, is determined based on the reaction rate between the lysine residues and dicarboxylic anhydride reactive groups. The number of non-nicotinamide-bonded, CDM-bonded lysine residues "c" can be selected as appropriate for the number of molecules of nicotinamide to be loaded into the micelles, and from the viewpoint of increasing the number of molecules of nicotinamide loaded, or from the viewpoint of increasing the hydrophobicity to improve the stability of the micelles, it is preferably 10 or greater, more preferably 20 or greater and even more preferably 30 or greater. From the viewpoint of stabilizing the micelles, the number of non-nicotinamide-bonded, CDM-bonded lysine residues "c" will usually be 60 or less, such as 40 or less.

The number of nicotinamide-bonded lysine residues "d" can likewise be selected as appropriate for the number of molecules of nicotinamide to be loaded into the micelles, and from the viewpoint of increasing the number of molecules of nicotinamide loaded, or from the viewpoint of increasing the hydrophobicity to improve the stability of the micelles, it is preferably 10 or greater, more preferably 20 or greater and even more preferably 30 or greater. The maximum for the number of nicotinamide-bonded lysine residues "d" is equal to the number of non-nicotinamide-bonded, CDM-bonded lysine residues "c" in the polyether-polyamino acid block copolymer before introduction of nicotinamide.

The total number of polyamino acid molecules can be set as desired depending on the number of polyether groups and the nature of the block copolymer, but as an example, it may be 2 to 80, and is preferably 20 to 60 or more preferably 30 to 50. The total of the number of non-CDM-bonded lysine residues "b", the number of non-nicotinamide-bonded, CDM-bonded lysine residues "c" and the number of nicotinamide-bonded lysine residues "d" in the nicotinamide-bonded polyether-polyamino acid block copolymer, as the final product, will not exceed the total number of polyamino acid groups. The total of the number of non-CDM-bonded lysine residues "b" and the number of non-nicotinamide-bonded, CDM-bonded lysine residues "c" in the polyether-polyamino acid block copolymer before introduction of nicotinamide, as the intermediate product, will also not exceed the total number of polyamino acid groups.

Nicotinamide bonds via the reactive groups bonded to the polyamino acid side chains of the polyether-polyamino acid block copolymer. The reactive groups may also bond via an additional linker. The reactive group type may be selected as desired in consideration of bondability with nicotinamide as the loading drug. An example for the reactive group is a dicarboxylic anhydride reactive group, such as carboxymethylmaleic anhydride (CDM), maleic anhydride or succinic anhydride. Dicarboxylic anhydride bonded to a lysine side chain reacts with the amine group of nicotinamide. As an example, a nicotinamide-bonded polyether-polyamino acid block copolymer may be produced by the reaction shown below. While nicotinamide may be bonded to all of the side chains to which carboxymethylmaleic anhydride is bonded, carboxymethylmaleic anhydride without bonding nicotinamide may remain, depending on the reaction rate. Carboxymethylmaleic anhydride may also be present as carboxymethylmaleic acid, depending on the surrounding environment. 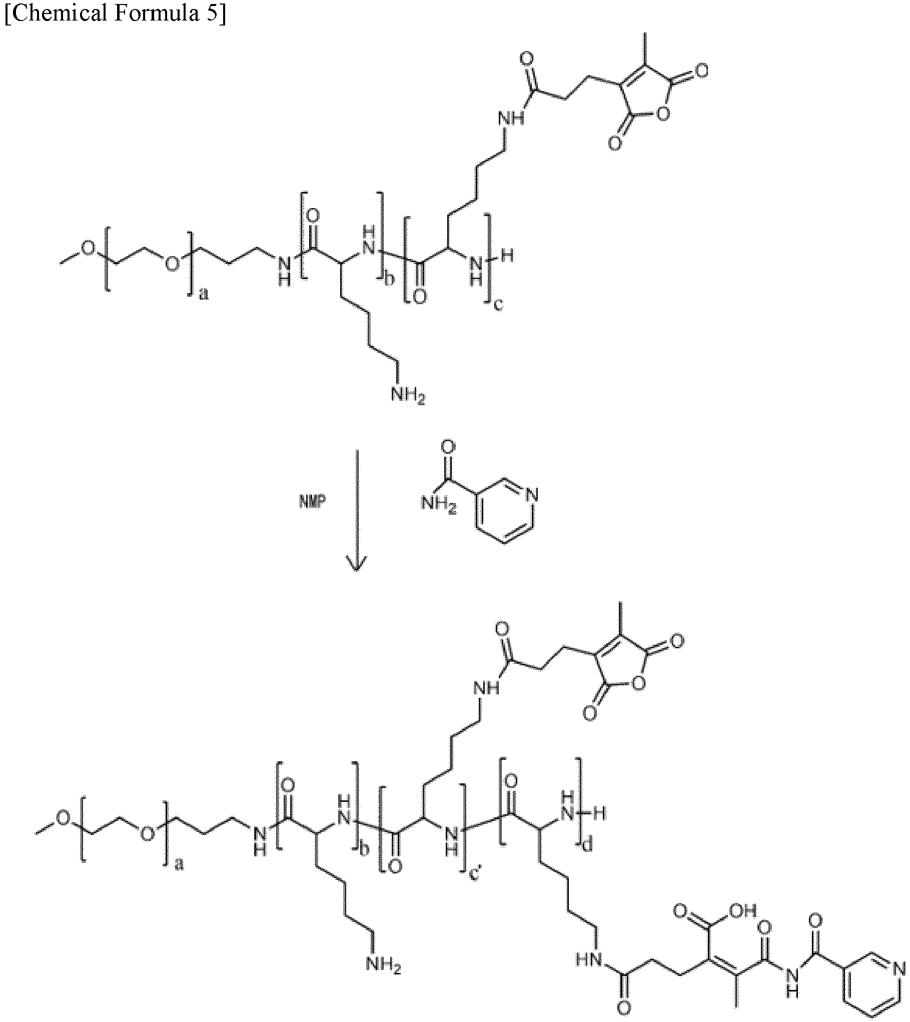 (In the formula, a = 45 to 2000, b = 0 to 20, c = c' + d, c' = 0 to 39, and d = 1 to 40.)

The introduction rate for nicotinamide loaded into the polyether-polyamino acid block copolymer used for the invention is not particularly restricted so long as nicotinamide-encapsulating micelles with the desired particle size can be prepared, but as an example it may be 60 to 90%, with an average of 10 to 20 molecules loaded per polymer. The nicotinamide will also sometimes be shed from the polymer during the process of preparing the micelles or during analysis. Therefore, the introduction rate for nicotinamide loaded into the polyether-polyamino acid block copolymer after micelle formation will be lower than the introduction rate before micelle formation. Assuming a usual reduction of 5 to 10%, the introduction rate for nicotinamide loaded into the polyether-polyamino acid block copolymer after micelle formation will therefore be 50 to 80%, with an average of 5 to 20 molecules loaded per polymer.

Another aspect of the invention relates to a method for producing nicotinamide-encapsulating micelles having a particle size of 25 to 100 nm. Specifically, the method includes:
a step of reacting nicotinamide with a polyether-polyamino acid block copolymer that includes a dicarboxylic anhydride reactive group on an amino acid side chain;
a step of purifying the nicotinamide-loaded polyether-polyamino acid block copolymer;
a step of dissolving the nicotinamide-loaded polyether-polyamino acid block copolymer in a polar solvent; and
a step of dialysis using a dialysis membrane with a molecular cutoff of 3500 to 16,000. Dialysis may also be carried out several times with different dialysis membranes to obtain micelles having a particle size of 25 to 100 nm, as measured by dynamic light scattering. Yet another aspect of the invention relates to nicotinamide-encapsulating micelles having a particle size of 25 to 100 nm, produced by the method described above.

As a preliminary step, the method for producing nicotinamide-encapsulating micelles having particle sizes of 25 to 100 nm according to the invention may also include a step of preparing a polyether-polyamino acid block copolymer including dicarboxylic anhydride reactive groups on the amino acid side chains. Specifically, it may include a step of mixing and reacting the polyether-polyamino acid block copolymer with an acyl chloride of dicarboxylic anhydride.

Reaction between nicotinamide and the dicarboxylic anhydride reactive groups may be conducted in any desired solvent, and as an example, it may be conducted in N-methyl-2-pyrrolidone, N,N-dimethylformamide, dimethyl sulfoxide, N,N-dimethyl acetamide, or a buffer solution.

Purification of the nicotinamide-loaded polyether-polyamino acid block copolymer is carried out by addition to an organic solvent such as diethyl ether or hexane to obtain a precipitate, and then recovery of the polymer by vacuum filtration.

The nicotinamide-loaded polyether-polyamino acid block copolymer forms micelles by dissolving in a polar solvent such as a buffer solution. The polymer micelles formed in this manner will generally aggregate as micelles with particle sizes of 20 nm to 100 nm. Purification may also be necessary by removal of the non-bonded polymer or drug. Ultrafiltration and dialysis are typical methods used for micelle purification. Dialysis is carried out against PBS, using a dialysis membrane with a molecular cutoff of 3500 to 16,000, for example. Dialysis allows removal of the polymer or drug that has not formed micelles, as well as micelles of different sizes than the desired size. Micelles of the desired particle size can also be obtained by ultrafiltration using a filter matching the desired micelle particle size. The filter used for ultrafiltration may be a filter with a molecular cutoff of 1000 to 100,000, as one example. Carrying out dialysis and/or ultrafiltration several times will allow micelles with a lower dispersity to be obtained.

The introduction rate for nicotinamide with respect to the polyether-polyamino acid block copolymer forming the micelles can be determined by ¹H-NMR after freeze-drying a solution of the micelles. The quality of the micelles can be adjusted by setting the particle sizes of the micelles based on dynamic light scattering, and also the dispersity according to gel permeation chromatography. The micelles of the invention preferably have particle sizes of 30 to 50 nm, and a dispersity of 0.10 to 0.30.

The nicotinamide-encapsulating micelles formed from the nicotinamide-loaded polyether-polyamino acid block copolymer obtained after purification may be used either directly or after sterilization if necessary, with optional addition of auxiliary agents suited for formulation, to prepare a medical formulation. The medical formulation may be prepared as an injection or infusion. A solution of the nicotinamide-encapsulating micelles may also be freeze-dried as a solid powder. A powder can also be administered after reconstitution with a dosable solution. The dosable solution used may be deionized water, or a buffer solution adjusted to a fixed pH.

The nicotinamide-encapsulating micelles and micelle-containing composition of the invention can be administered to a subject suffering from hypertension during pregnancy. More specifically, the subject is a subject suffering from gestational hypertension syndrome. The micelles may be administered to the subject by any parenteral administration route such as intravenous, subcutaneous or intramuscular administration, but in most cases it will be administered as an intravenous injection or bolus injection.

### EXAMPLES

### Example 1: Production of nicotinamide-encapsulating micelles

### (1) Preparation of polyethylene glycol-poly(lysine) block copolymer (PEG-p(Lys))

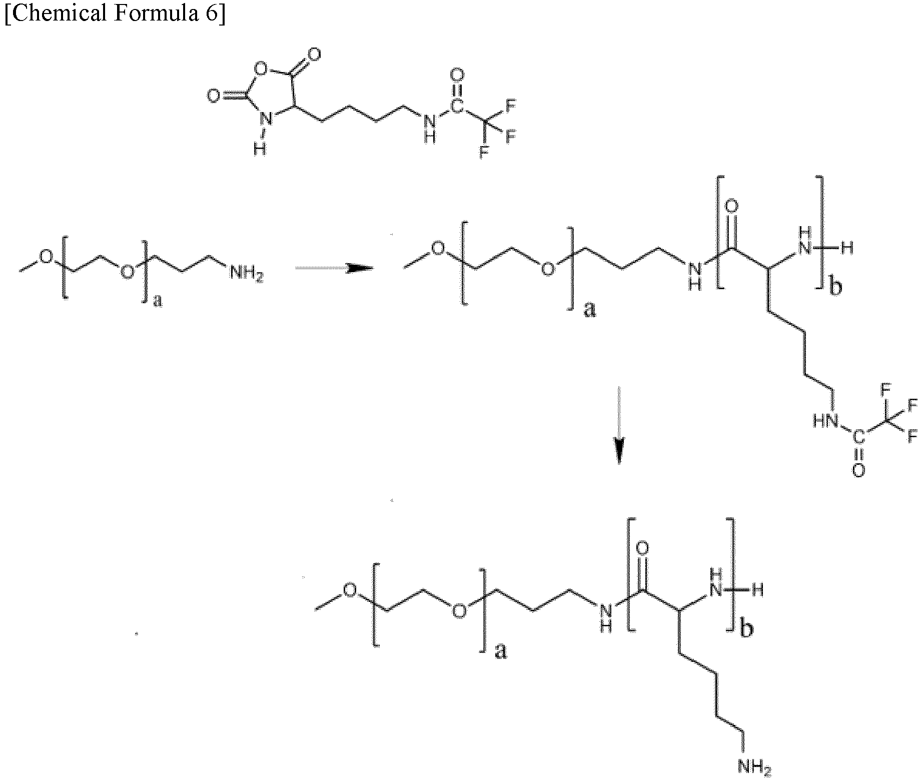

(In the formulas, "a" and "b" are the molecular weight of PEG used and the number of reactions of Lys(TFA)-NCA, respectively.)

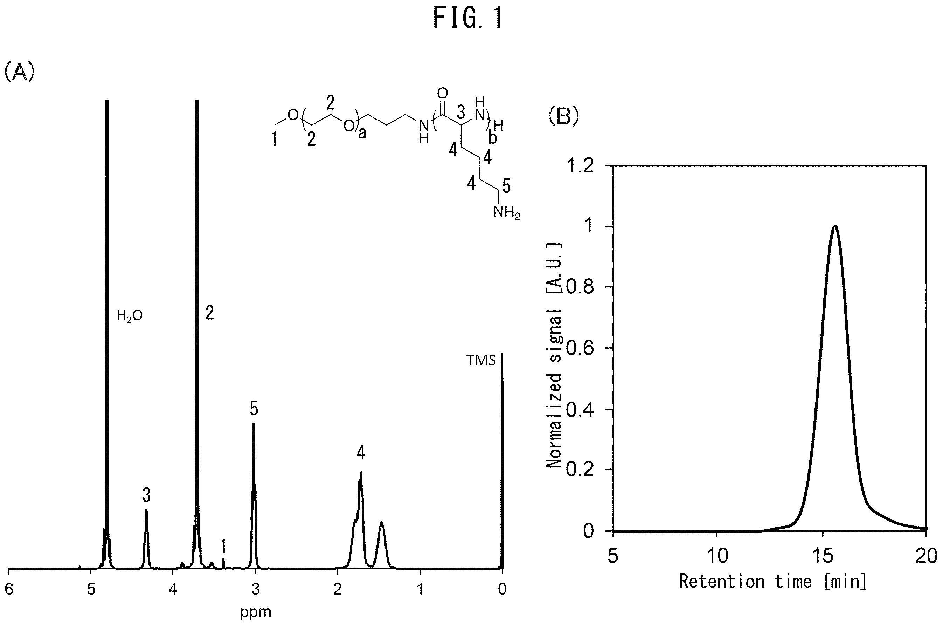

PEG-p(Lys) was prepared by a previously reported method (NPL 2:Macromol. Biosci. (2020) (20) 1900161). Specifically, α-methoxy-ω-amino-poly(ethylene glycol) (MeO-PEG-NH₂; Mw = 12,000 g/mol) (NOF Corporation) and N-trifluoroacetyl-L-lysine N-carboxyanhydride (Lys(TFA)-NCA) (Chuo Kaseihin Co., Inc.) were provided for ring-opening polymerization reaction, forming polyethylene glycol-poly(trifluoroacetyl-lysine) (PEG-p(Lys-TFA)), after which the trifluoroacetyl groups were removed by deprotection. More specifically, thiourea (1.5 g, 20 mmol) was dissolved in dehydrated DMF (20 ml) to obtain thiourea-containing DMF. Next, MeO-PEG-NH₂ (1 g, 0.083 mmol) and Lys(TFA)-NCA (1.005 g, 3.75 mmol) were dissolved in the thiourea-containing DMF (10 ml), and the mixture was stirred for reaction for 3 days at 35°C under an argon atmosphere. The reaction mixture was precipitated in diethyl ether and dried by suction to obtain a polymer as a white powder. The polymerization degree was determined using an ¹H-NMR spectrometer (DMSO-d₆, 80°C), and the molecular weight distribution was measured by organic phase GPC (eluate: 10 mM LiCl-containing DMF; temperature: 40°C; flow rate: 0.8 ml/min; detector: refractive index). The trifluoroacetyl protecting groups were also removed by treatment overnight with a 1 N NaOH-methanol solution at 35°C, and dialysis was carried out with a 6 to 8 kD MWCO dialysis membrane. After freeze-drying, the final product was obtained as a white powder. The deprotected polymer was analyzed using a ¹H-NMR spectrometer (D₂O, 25°C). The composition of the PEG-p(Lys) block copolymer was identified using the proton peaks for the -OCH₂-CH₂- of PEG and -C₃H₆ of lysine. The molecular weight distribution was analyzed by aqueous phase GPC (mobile phase: pH 3.3 acetate-buffered saline (10 mM acetate and 500 mM-NaCl); room temperature; flow rate: 0.75 ml/min; detector: UV; wavelength: 220 nm). The NMR data and GPC data are shown in Fig. 1.

### (2) Preparation of polyethylene glycol-poly(lysine-carboxymethylmaleic anhydride) block copolymer (PEG-p(Lys-CDM))

An acyl chloride of carboxymethylmaleic anhydride (CDM) was reacted with PEG-p(Lys) to obtain PEG-p(Lys-CDM). 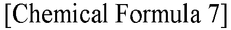 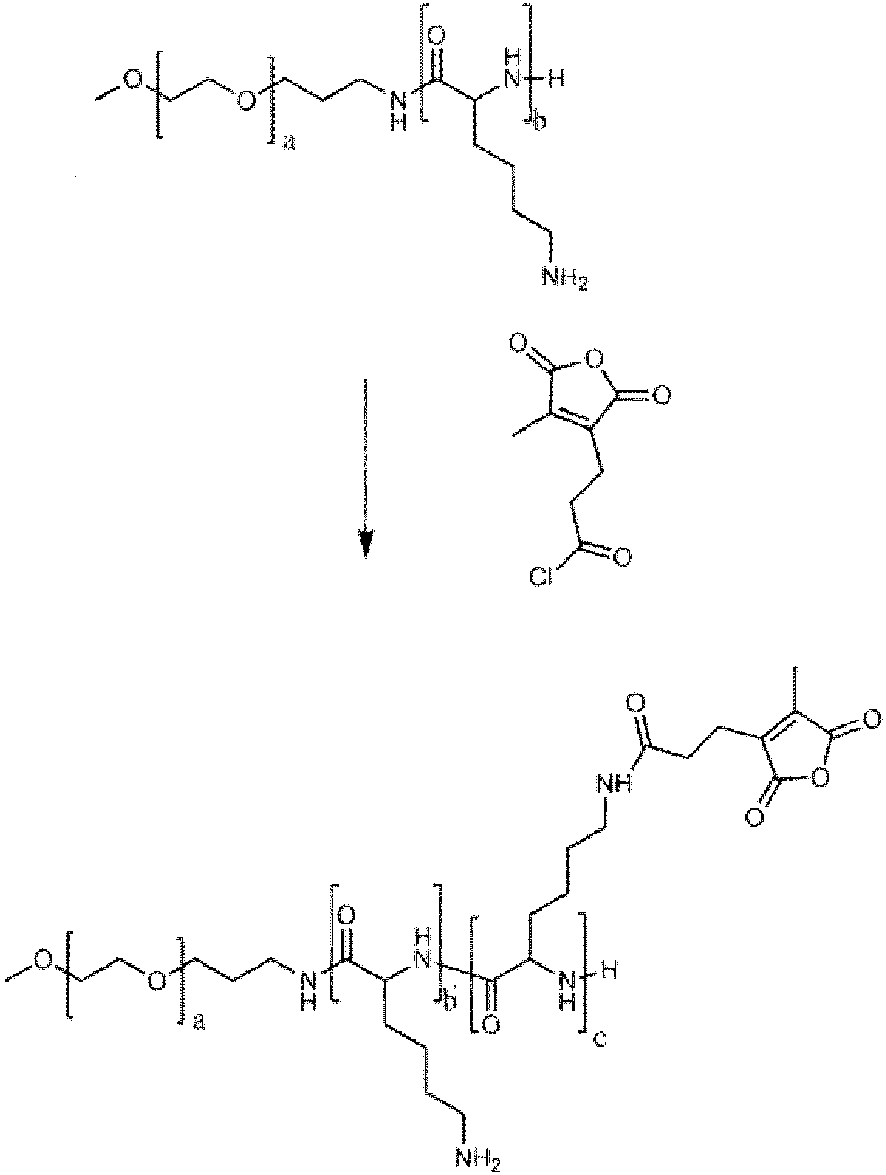 (In the formulas, "a" depends on the molecular weight of the PEG used, "b" depends on the number of Lys(TFA)-NCA reactions, and "c" depends on the number of CDM reactions, with the condition that b' = b - c.)

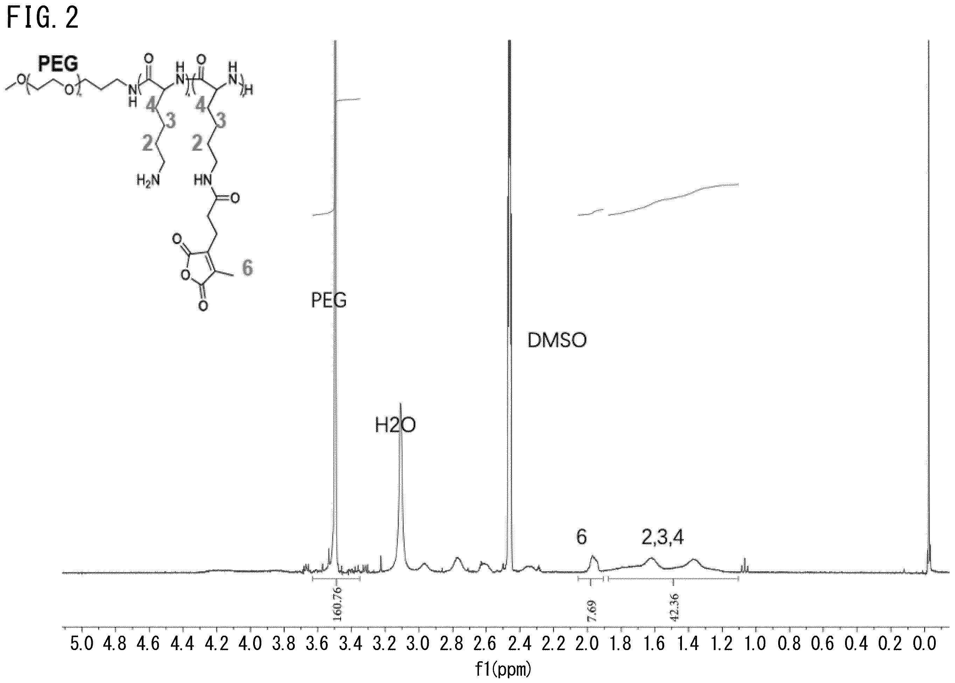

After dissolving 2-propionic-3-methylmaleic anhydride (CDM, Tokyo Chemical Industry Co. Ltd., 200 mg, 1.09 mmol) in anhydrous toluene, the mixture was evaporated by suction. The CDM was dissolved in anhydrous CH₂Cl₂ (15 ml), and then oxalyl chloride (Tokyo Chemical Industry Co. Ltd., 4 ml, 5.9 g, 46 mmol) was added and reaction was conducted for 12 hours at room temperature. The CH₂Cl₂ and residual oxalyl chloride were then removed by evaporation to obtain a transparent oil. After then adding CH₂Cl₂ (4 ml) to dissolve the CDM-Cl, CH₂Cl₂ (20 ml) was used to dissolve the PEG-P(Lys) (200 mg, 0.011 mmol). The PEG-p(Lys) solution was then transferred to the CDM-Cl solution and the reaction mixture was stirred for 12 hours at room temperature. After stirring, diethyl ether precipitation and overnight suction drying were carried out to obtain a product. The product was analyzed by 1H NMR and confirmed to include 20 non-CDM-bonded lysines and 17 CDM-bonded lysines per polymer. The NMR data are shown in Fig. 2.

### (3) Production of nicotinamide-bonded polyethylene glycol-polyamino acid block copolymer (PEG-p(Lys-CDM-NA))

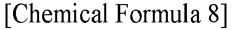

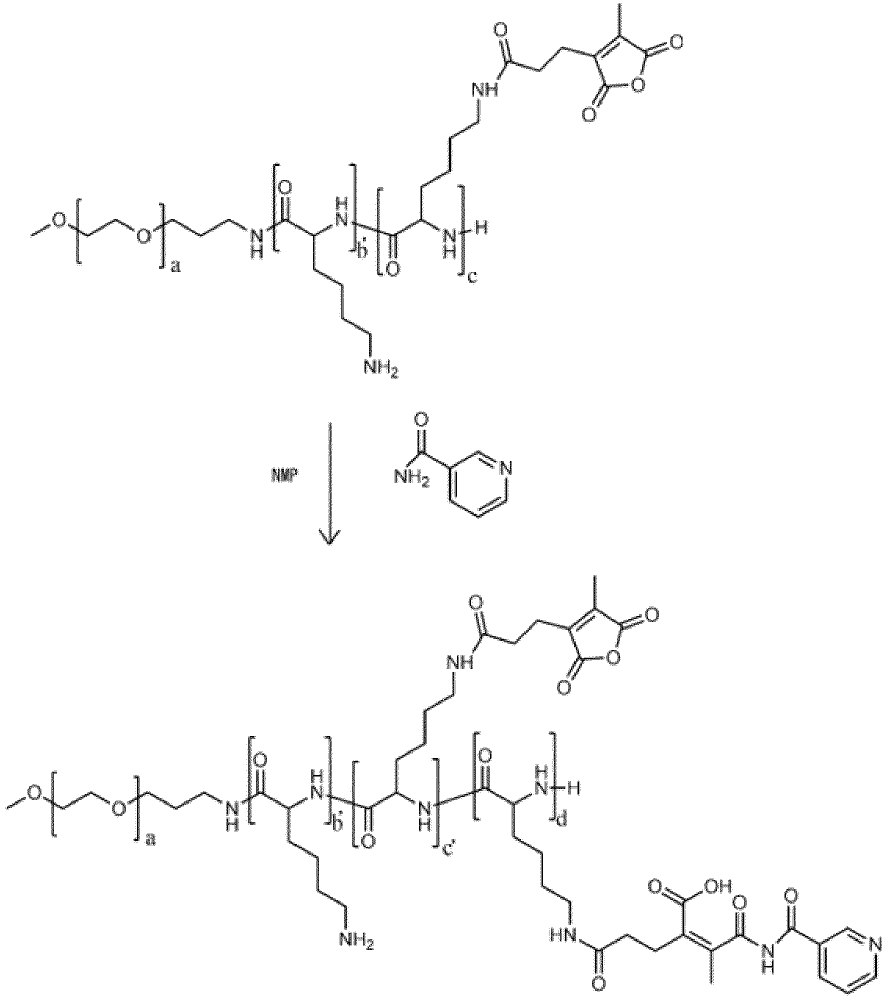

(In the formulas, "a" depends on the molecular weight of the PEG used, b' = b - c, where "b" depends on the number of Lys(TFA)-NCA reactions and "c" depends on the number of CDM reactions, and c' = c - d, where "d" depends on the number of nicotinamide reactions.)

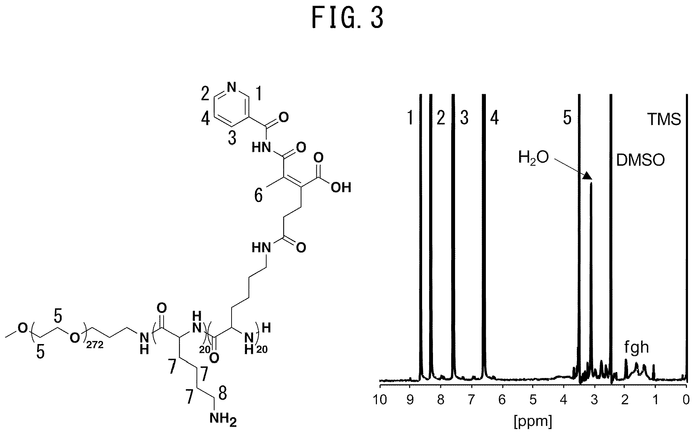

PEG-p(Lys-CDM) (10 mg) and nicotinamide (distributor: Tokyo Kasei Kogyo Co., Ltd., 10-fold amount with respect to CDM: 12 mg) were reacted for 24 hours at room temperature in N-methyl-2-pyrrolidone (NMP: 2 mL). The reaction mixture was added to diethyl ether, forming a precipitate. The polymer was recovered by filtration under reduced pressure. The recovered polymer was analyzed by 1H NMR and the introduction rate for nicotinamide was found to be 73% with respect to CDM in the polymer (15 per polymer). The NMR data are shown in Fig. 3.

### (4) Production of micelles

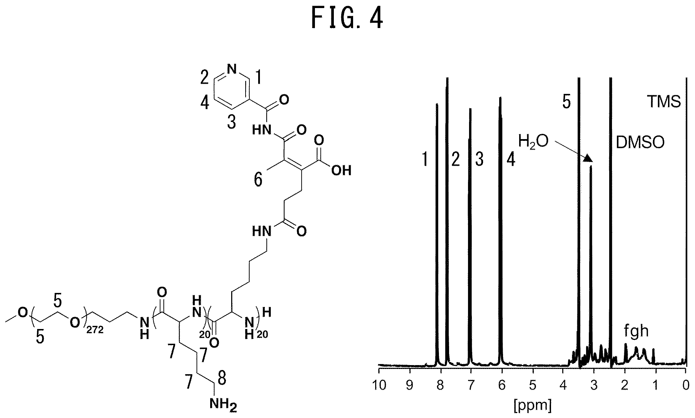

Nicotinamide-encapsulating micelles (PEG-p(Lys-CDM-NA) micelles) were prepared by dialysis. The PEG-p(Lys-CDM-NA) polymer was dissolved in N-methylpyrrolidone (1 mg/mL), and purified by dialysis against PBS (pH 7.4) using a Por 3 Dialysis Membrane (MWCO: 3500) (24 hours, 6 times total). The sizes and polydispersity index (PDI) of the prepared micelles were measured by dynamic light scattering (DLS). The micelle sizes were 42 nm and the polydispersity index was 0.19. The polymer in the micelles was recovered by freeze-drying, and the nicotinamide introduction rate was determined by 1H-NMR (65%; 13 per polymer). The NMR data are shown in Fig. 4.

### Example 2: Property analysis of nicotinamide-encapsulating micelles

### (1) pH-dependent drug release test

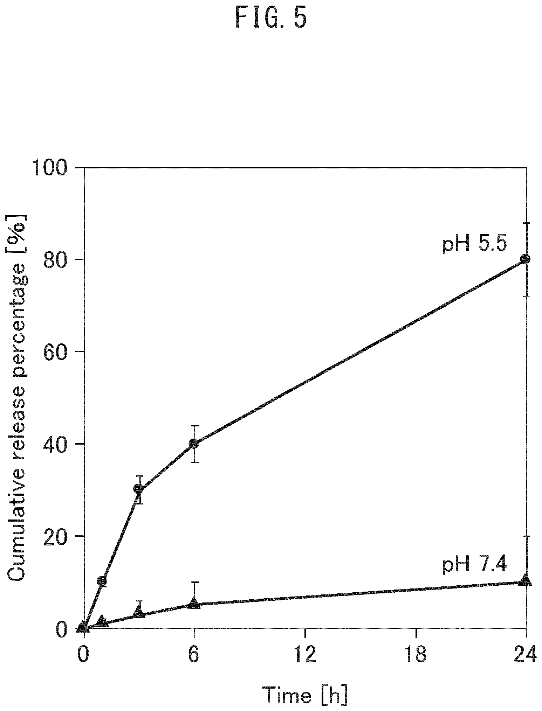

The micelle solution was dialysed against PBS (pH 7.4) and PBS (pH 6.5) using a Por 3 Dialysis Membrane (MWCO: 3500). The external solution was sampled at 1 hour, 3 hours, 6 hours and 24 hours after the start of dialysis. The sampled external solution was concentrated and then freeze-dried. The powder was dissolved and quantified by HPLC (268 nm UV, mobile phase: 20 mM phosphate buffer). The results are shown in Fig. 5.

### Example 3: Evaluating accumulation in placenta and fetus using polymer micelles of different sizes

For this Example, fetal and placental accumulation of polymer micelles and low molecular compounds of different sizes was evaluated by fluorescence analysis, elemental analysis and mass spectrometry using pregnancy model mice.

### (1) Preparation of DACH-platin encapsulating micelles

DACH-platin encapsulating micelles were prepared as two fluorescently labeled types (30 nm and 70 nm particle sizes, Alexa-555 labeling for 30 nm-particle size micelles, Alexa647 labeling for 70 nm-particle size micelles) (NPL 3: H. Cabral et. al., Nat. Nanotech. 6(2011) 815-823).

### (2) Administration to pregnancy model mice

Mixed solutions of the two DACH-platin encapsulating micelle types (30 nm and 70 nm particle sizes) were administered to mice on the 14th day after pregnancy. At 48 hours after administration, the placentas and fetuses were extracted and frozen samples were prepared. Frozen sections (thickness: 10 mm) were then prepared and nuclear stained with Hoechst. The Hoechst-fluorescence (Fig. 11A) and micelle labeling (Alexa 555 and Alexa647) fluorescence (Figs. 11B and C) were measured. The results in Fig. 11 confirmed that the two types of DACH-platin encapsulating micelles (particle sizes of 30 nm and 70 nm) had not accumulated in the fetus (F). With the DACH-platin encapsulating micelles of 30 nm particle size, no accumulation was seen in the fetus (F), but the micelles accumulated up to the labyrinth (L) layer on the fetus side of the placenta. With the DACH-platin encapsulating micelles of 70 nm particle size, accumulation was seen in the decidua (D) layer on the maternal side of the placenta, but no accumulation was seen in the spongiotrophoblast (S) layer between the D layer and L layer. These results indicated that intraplacental distribution can be controlled by precise design of drug size.

### (3) Evaluation of permeability through human placenta

For this Example, the permeability of polymer micelles, low molecular compounds and polymers of different sizes through the human placenta was evaluated using a human placental perfusion model.

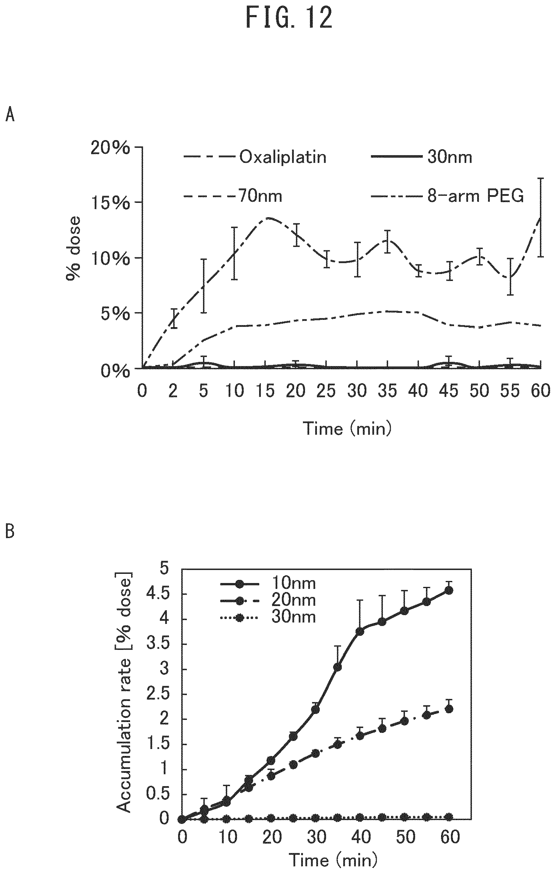

The human placental perfusion model was created according to NPL 4 (K. Shintaku et. al., Drug Metab. Dispos. 37 (2009) 962) and NPL 5 (J. R. Huston et. al., Clin. Pharmacol. Ther. 90 (2011) 67). Specifically, a needle (18 gauge) was introduced into the maternal side and a needle (18 gauge) was introduced into the vein and artery on the fetus side of a human placenta provided from the University of Tokyo Hospital, Dept. of Obstetrics and Gynecology, and Krebs-Ringer Bicarbonate Buffer prepared according to NPL 6 (M. Nagai et. al., Drug Metab. Dispos. 41 (2013) 2124) was perfused through at 37°C for 30 minutes (flow rate on maternal side: 15 mL/min, flow rate on fetus side: 3 ml/min). The buffer was neutralized using oxygen produced by oxidation-reduction reaction of hydrogen peroxide water and iron chloride. DACH-platin encapsulating micelles (30 nm and 70 nm particle sizes) (DACH-platin dose: 85.4 mg/L), oxaliplatin (2.7 mg/L) and Cy3-labeled 8-arm PEG (fluorescence intensity: 3,000 (RFU)) were perfused for 60 minutes, and 1 mL of each solution was recovered every 5 minutes from the vein on the fetus side. The platinum contents of the recovered samples from the oxaliplatin- and DACH-platin encapsulating micelle-administered groups were measured by inductively coupled plasmon mass spectrometry. The fluorescence intensities of the polymers were quantified by HPLC. The ratio of permeation volume with respect to dose of each agent was calculated, as shown in Fig. 12A. The results shown in Fig. 12A indicate that oxaliplatin permeated the placenta, but that the DACH-platin encapsulating micelles (30 nm and 70 nm particle sizes) both failed to permeate the placenta, thus suggesting that permeation through the placenta was inhibited by using a drug size of 30 nm or greater. Permeation through the placenta was also seen with 8-arm PEG, though to a lesser degree than oxaliplatin, suggesting a drug size threshold of between 10 nm and 30 nm. Since the results in Fig. 12A showed accumulation of oxaliplatin in the placenta but no accumulation of micelles in the placenta, it was suggested that accumulation of micelles in the placenta was inhibited by differences in the mouse and human placenta structures. It was thus confirmed that accumulation in and permeation through the placenta can be inhibited by using a drug size of 30 nm or greater.

In order to identify the sizes of the substances passing through the placenta, 10, 20 and 30 nm PEG-coated gold nanoparticles (NP) (5 mL, Nanocs, Inc., USA) were used for an *ex vivo* human placenta perfusion experiment in the same manner as described above. With perfusion of the 10 nm Gold NP, detection of gold nanoparticles from the fetus side increased in a time-dependent manner, reaching 4.58% of the administered amount after 60 minutes. With perfusion of 20 nm Gold NP, detection from the fetus side was 2.21%. With perfusion of 30 nm Gold NP, detection from the fetus side was 0.05% of the administered dose after 60 minutes. The results are shown in Fig. 12B. These results suggest that a size of between 20 nm and 30 nm may be the cut-off for passage of materials through the placenta.

### Example 4: Evaluating accumulation in placenta and fetus using nicotinamide-encapsulating micelles

### (1) Administration to pregnancy model mice

The nicotinamide-encapsulating micelles produced in Example 1 were administered to pregnancy model mice in the same manner as Example 3(2), and their degree of accumulation in the placenta and fetus was evaluated.

### (1-1) Evaluation of blood retentivity

Nicotinamide-encapsulating micelles (micelle size: 42 nm, polydispersity index: 0.19, nicotinamide introduction rate: 65%, 10 mg nicotinamide encapsulation in micelles) were dissolved in PBS, and the solution was intravenously administered through the caudal vein of mice on the 17th day after pregnancy. As a control, 10 mg of nicotinamide dissolved in PBS was intravenously administered through the caudal vein of mice on the 17th day after pregnancy. Blood was sampled from the eye bulb at 1 hour, 3 hours, 6 hours, 24 hours and 48 hours following administration. The pH of the harvested blood sample was adjusted, releasing the nicotinamide in the micelles, and the amount of nicotinamide in the blood sample was measured by HPLC (detector: EXTREMA by JASCO Corp., column:: TSKgel ODS-120H by Tosoh Corp., 1.9 µm). This change in the blood concentration of nicotinamide compared to 100 as the concentration of the nicotinamide dilution before administration is shown (Fig. 7). Nicotinamide decreased to about 1/10 by 6 hours after administration, but with the nicotinamide-encapsulating micelles it decreased to about 1/10 at 48 hours after administration, and therefore retentivity in the blood increased.

### (1-2) Evaluating accumulation in placenta and fetus

Nicotinamide-encapsulating micelles (micelle size: 42 nm, polydispersity index: 0.19, nicotinamide introduction rate: 65%, 10 mg nicotinamide encapsulation in micelles) was dissolved in PBS, and the solution was intravenously administered through the caudal vein of mice on the 17th day after pregnancy. As a control, 10 mg of nicotinamide dissolved in PBS was intravenously administered through the caudal vein of mice on the 14th day after pregnancy. Mice were slaughtered before administration and 1 hour, 3 hours, 6 hours, 24 hours and 48 hours after administration, and the placenta and fetus were extracted. The placenta and fetus were each weighed and then homogenized and dissolved in PBS to prepare samples which were then adjusted in pH to release the nicotinamide in the micelles, and the nicotinamide concentrations in the samples were measured by HPLC (detector: EXTREMA by JASCO Corp., column:: TSKgel ODS-120H by Tosoh Corp., 1.9 µm). The percentage (%) with respect to the dose per 1 g of tissue was measured as the accumulated amount (Fig. 8). The nicotinamide-encapsulating micelles had reduced migration into the fetus, but accumulation in the placenta was found. Focusing on accumulation in the placenta, an immediate peak was produced after administration of nicotinamide, but the peak occurred after 24 hours with administration of nicotinamide-encapsulating micelles, resulting in a longer lasting effect and a higher accumulated amount in the placenta.

Using an osmotic pump attached subcutaneously to normal pregnant mice, nicotinamide-encapsulating micelles (micelle size: 42 nm, polydispersity index: 0.19, nicotinamide introduction rate: 65%, 10 mg nicotinamide encapsulated in micelles) dissolved in PBS were intravenously administered through the caudal vein to hypertension model mice after 17 days of pregnancy. Separately, 10 mg of nicotinamide dissolved in PBS was intravenously administered through the caudal vein of similar hypertension model mice. Mice were slaughtered before administration and 1 hour, 3 hours, 6 hours, 24 hours and 48 hours after administration, and the placentas were extracted. Each placenta was weighed and then homogenized and dissolved in PBS to prepare samples which were then adjusted in pH to release the nicotinamide in the micelles, and the nicotinamide concentrations in the samples were measured by HPLC (detector: EXTREMA by JASCO Corp., column:: TSKgel ODS-120H by Tosoh Corp., 1.9 µm). The percentage (%) with respect to the dose per 1 g of tissue was measured as the accumulated amount (Fig. 10(1)). Fig. 10(2) shows accumulated amount of nicotinamide in the placenta when the same experiment was conducted on mice on the 17th day of normal pregnancy. When nicotinamide-encapsulating micelles were administered to hypertension model mice, nicotinamide accumulation in the placenta was higher compared to the normal mice.

### (2) Evaluation of permeability through human placenta

The nicotinamide-encapsulating micelles produced in Example 1 were evaluated for permeability through the placenta and degree of accumulation in the placenta using a human placental perfusion model, in the same manner as Example 3(3).

The human placental perfusion model was created according to NPL 4 (K. Shintaku et. al., Drug Metab. Dispos. 37 (2009) 962) and NPL 5 (J. R. Huston et. al., Clin. Pharmacol. Ther. 90 (2011) 67). Specifically, a needle (18 gauge) was introduced into the maternal side and a needle (18 gauge) was introduced into the vein and artery on the fetus side of a human placenta provided from the University of Tokyo Hospital, Dept. of Obstetrics and Gynecology, and Krebs-Ringer Bicarbonate Buffer prepared according to NPL 6 (M. Nagai et. al., Drug Metab. Dispos. 41 (2013) 2124) was perfused through at 37°C for 30 minutes (flow rate on maternal side: 15 mL/min, flow rate on fetus side: 3 ml/min). The buffer was neutralized using oxygen produced by oxidation-reduction reaction of hydrogen peroxide water and iron chloride. Next, nicotinamide-encapsulating micelles (micelle size: 42 nm, polydispersity index: 0.19, nicotinamide introduction rate: 65%, 100 mg nicotinamide encapsulation in micelles) dissolved in 1 L of PBS were perfused for 60 minutes, recovering 1 mL of the solution from the vein on the fetus side every 10 minutes. As a control, 100 mg of nicotinamide dissolved in 1 L of PBS was perfused for 60 minutes in the same manner, and solution was recovered from the vein on the fetus side. The pH of the recovered sample was adjusted, releasing the nicotinamide, and the nicotinamide in the solution was measured by HPLC (detector: EXTREMA by JASCO Corp., column: TSKgel ODS-120H by Tosoh Corp., 1.9 µm). The percentage of the nicotinamide concentration in the solution after placental passage with respect to the nicotinamide concentration in the solution before placental passage was recorded as the placental permeability (Fig. 6).

### Example 5: Evaluation of therapeutic effect on hypertension using nicotinamide-encapsulating micelles

Angiotensin II (distributor: Sigma-Aldrich, 1.5 µg/kg mouse) was administered subcutaneously to normal pregnant mice (10th day of pregnancy) using a subcutaneously attached osmotic pump (Day 0) until the 17th day of pregnancy (Day 7), to produce pregnancy hypertension model mice. The drug was intravenously administered through the caudal vein from the 13th day (Day 3) until the 16th day (Day 6) of pregnancy. The administered drugs were nicotinamide (10 mg/day) and nicotinamide-encapsulating micelles (micelle size: 42 nm, polydispersity index: 0.19, nicotinamide introduction rate: 65%, 10 mg nicotinamide encapsulation in micelles), each dissolved in PBS. Drug-free PBS (PBS) was administered as a control. An angiotensin II non-administered group was also used as a control (Control). The systolic blood pressure of the mice was measured on Day 0, 3, 5 and 7 (Fig. 9). Administration of angiotensin II increased blood pressure (Day 3), whereas the groups administered nicotinamide and nicotinamide-encapsulating micelles had decreased blood pressure (Day 5 and Day 7). The blood pressure lowering effect was higher with administration of nicotinamide-encapsulating micelles compared to administration of nicotinamide.

## Claims

1. Nicotinamide-encapsulating micelles formed from a polyether-polyamino acid block copolymer, wherein nicotinamide is bonded to an amino acid side chain, and wherein the particle size is 25 to 100 nm as measured by dynamic light scattering.

2. The micelles according to claim 1, wherein the polyether-polyamino acid block copolymer comprises lysine as an amino acid block.

3. The micelles according to claim 1 or 2, wherein the polyether-polyamino acid block copolymer is a polyethylene glycol-polyamino acid block copolymer.

4. The micelles according to any one of claims 1 to 3, wherein the nicotinamide is bonded to the amino acid side chain via a dicarboxylic acid anhydride reactive group.

5. The micelles according to any one of claims 1 to 4, wherein the polyether-polyamino acid block copolymer is a compound of the following formula: 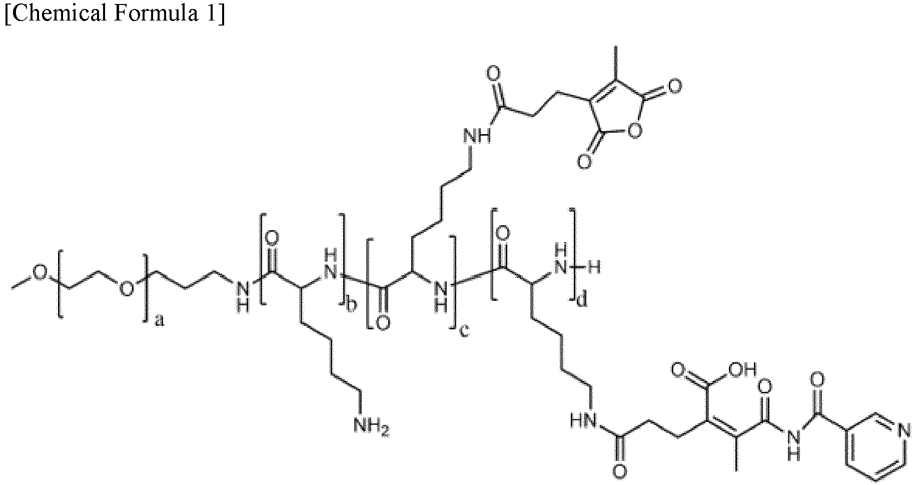 (wherein a = 45 to 2000, b = 0 to 20, c = 0 to 39 and d = 1 to 40).

6. The micelles according to any one of claims 1 to 5, wherein the particle sizes of the nicotinamide-encapsulating micelles are 30 to 50 nm.

7. A composition containing micelles according to any one of claims 1 to 6.

8. The composition according to claim 7, wherein the composition is a pharmaceutical composition for treatment or prevention of hypertension during pregnancy.

9. The composition according to claim 8, wherein the composition is a pharmaceutical composition for treatment or prevention of hypertension during pregnancy which is gestational hypertension syndrome and includes miscarriage, premature birth and/or intrauterine fetal growth retardation caused by gestational hypertension syndrome.

10. The composition according to any one of claims 7 to 9, wherein the migration to the fetus is reduced.

11. The composition according to any one of claims 7 to 10, wherein the composition is a placenta-targeted composition.

12. A method for producing nicotinamide-encapsulating micelles for treatment of pregnancy hypertension, wherein the method comprises:
reacting nicotinamide with a polyether-polyamino acid block copolymer that includes a carboxymethylmaleic anhydride-reactive group on an amino acid side chain;
purifying the nicotinamide-loaded polyether-polyamino acid block copolymer;
dissolving the nicotinamide-loaded polyether-polyamino acid block copolymer in a polar solvent; and
ultrafiltration using a dialysis membrane with a molecular cutoff of 3500 to 16,000 to obtain micelles having a particle size of 25 to 100 nm as measured by dynamic light scattering.

13. The method according to claim 12, wherein the polyether-polyamino acid block copolymer that includes a carboxymethylmaleic anhydride-reactive group on an amino acid side chain is represented by the following formula: 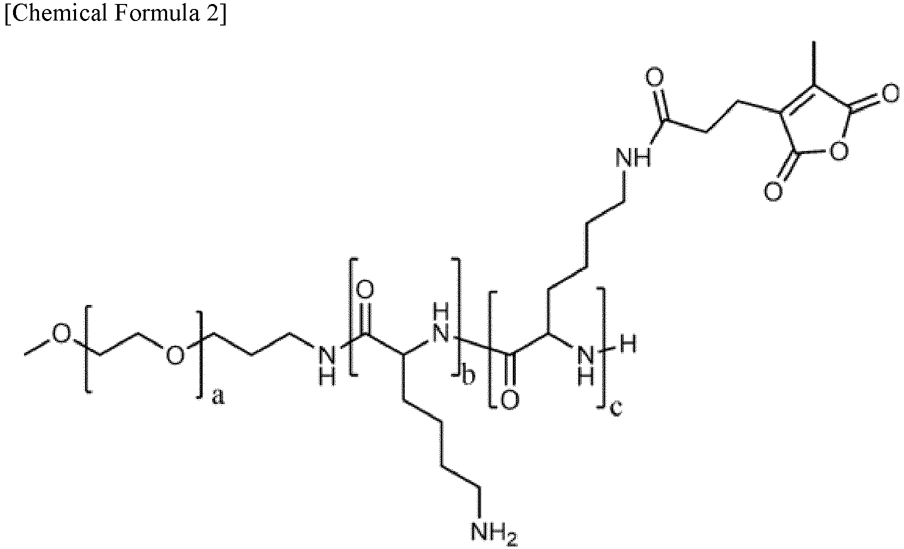 (wherein a = 45 to 2000, b = 0 to 20 and c = 1 to 60).
